# EUROPEAN PATENT APPLICATION

(11) **EP 4 176 898 A1**
(43) Date of publication of application: **10.05.2023**
(21) Application number: 22170226.9
(22) Date of filing: 27.04.2022
(51) Int. Cl.: A61K 39/12, A61P 31/14, A61K 39/00

(54) **PAN SARS-COV2 VACCINE ANTIGEN**

(30) Priority: 08.11.2021 EP 21206845
(71) Applicant: Charité - Universitätsmedizin Berlin, 10117 Berlin (DE)
(72) Inventor: SCHÖNRICH, Günther, 10781 Berlin (DE); YASSEN, Mohammed, 10319 Berlin (DE); RAFTERY, Martin, 61352 Bad Homburg (DE)
(74) Representative: Schulz Junghans Patentanwälte PartGmbB

(57) **Abstract**

In one aspect, the invention relates to a Sars-Cov-2 vaccine antigen amino acid sequence comprising a plurality of each of: nonstructural protein T cell epitopes, accessory protein T cell epitopes, spike protein T cell epitopes, envelope protein T cell epitopes, membrane protein T cell epitopes, nucleocapsid protein T cell epitopes.

## Description

The present invention relates to a vaccine antigen amino acid sequence for vaccination or therapy of SARS-CoV-2 infection, comprising consensus T cell epitopes representing the SARS-CoV-2 S (spike) protein, E (envelope) protein, N (nucleocapsid) protein, M (membrane) protein, nonstructural proteins (NSP), and accessory proteins, and further to a nucleic acid sequence encoding such antigen.

### Background of the Invention

Coronaviruses comprise a large family of enveloped, positive-sense single-stranded RNA viruses within the order *Nidovirales.* They are linked to diseases in birds and mammals. Several strains within the genera alpha- and betacoronaviruses are known to cause common cold in humans: the alphacoronaviruses HCoV-229E and HCoV-NL63 and the betacoronaviruses HCoV-OC43 and HCoV-HKU1. Since the outbreak of severe acute respiratory syndrome (SARS) in 2002 in Guangdong province, China, three betacoronaviruses causing severe disease in humans have been discovered: Middle East respiratory syndrome coronavirus (MERS-CoV), SARS-CoV, and SARS-CoV-2.

There is ample evidence that all known recently emerged human coronaviruses originated in bats. SARS-CoV and MERS-CoV were responsible for separate viral epidemics with case fatality rates of up to 10% and 35%, respectively. After it emerged in Asia 2019, SARS-CoV-2 rapidly spread throughout the globe.

SARS-CoV-2 is a serious threat for global health because it can cause coronavirus disease 2019 (COVID-19). The incubation period for SARS-CoV-2 infection can extend to 14 days, with a median time of 4-5 days from exposure to debut of symptoms. Acute infection starts with viral replication in the upper respiratory tract and can be asymptomatic. A symptomatic course often shows only mild symptoms (sore throat, cough, fever, fatigue, myalgia, headache, and diarrhea). Severe COVID-19 usually begins approximately 1 week after onset of symptoms and is associated with SARS-CoV-2 infection of the lower respiratory tract. This can result in acute respiratory distress syndrome (ARDS). Frequently, lymphopenia and thromboembolic complications occur. Moreover, acute cardiac, kidney, and liver failure followed by coagulopathy and shock may be critical. Despite radical measures to stop the pandemic, a substantial number of individuals developed severe COVID-19, causing an estimated number of fatalities exceeding 4 million by October 2021.

At least two other different periods of disease have been linked to SARS-CoV-2 infection: a rare post-acute multisystem inflammatory syndrome (2-5 weeks after onset of symptoms), and late inflammatory sequelae (Long-COVID-19) such as chronic fatigue and loss of sense of smell taste. Even individuals with mild COVID-19 can suffer from variable and debilitating symptoms for many months after the initial infection. In the face of an ongoing pandemic with hundreds of millions of confirmed COVID-19 cases, even a small incidence of Long-COVID-19 with ca. 10% of infected people will have dramatic consequences for economy and health care systems.

Mass vaccination campaigns are key in the prophylaxis of COVID-19 and regarded as the only realistic scenario to end strict social isolation measures and concomitant economic shutdown. Even if these efforts will be successful, it is very likely that the virus is here to stay as an endemic pathogen making safe and efficient vaccination also mandatory in the future. Current COVID-19 vaccines comprise virally vectored platforms (replicating or non-replicating), DNA- and mRNA-based vaccines, alum-adjuvanted inactivated virus, and recombinant protein [Krammer, Nature, 2020. 586(7830): p. 516-527.; Kyriakidis et al., npj Vaccines, 2021. 6(1): p. 28.]. Similar to virus infections, vaccines cannot only stimulate B cells to produce virus-specific antibodies but also CD4+ T cells and CD8+ T cells. CD4+ T cells possess a range of helper and effector functionalities whereas CD8+ T cells called cytotoxic T cells (CTLs) kill virus-infected cells.

At this stage, however, all approved vaccines against COVID-19 and almost all of those in clinical trials are designed to induce preferentially neutralizing antibodies (nAbs) [Krammer, ibid; Kyriakidis, ibid., Dai and Gao, Nature Reviews Immunology, 2021. 21(2): p. 73-82.; Jeyanathan et al., Nature Reviews Immunology, 2020. 20(10): p. 615-632, Rapaka et al., Clinical Infectious Diseases, 2021.]. These are directed against the spike (S) protein of SARS-CoV-2, which mediates viral entry into host cells via the main viral receptor, angiotensin converting enzyme-2 (ACE2). The approved vaccines from Pfizer-BioNTech and Moderna are based on S-protein-encoding mRNA delivered by lipid nanoparticles (LNPs), while the approved formulations by AstraZeneca, Johnson and Johnson and Gam-COVID-vac (Sputnik V) contain S-protein-encoding DNA delivered within non-replicating recombinant adenovirus (AdV) vector systems. Both, mRNA and AdV vaccines, possess inherent adjuvant activity, which stimulates the innate immune system and provides the required second signal for T cell activation.

Explaining the strategic decision for S-protein-based vaccines, nAbs targeting the S protein can prevent infection ("sterilizing immunity"), if produced in large quantities by the immune system. Most nAbs from COVID-19 convalescent patients target the receptor-binding domain (RBD) or the N-terminal domain (NTD) of the S protein. Some nAbs derived from patients are already in clinical use as prophylactic and therapeutic agents. Importantly, the globally applied vaccines and nAbs are all based on the first available S-protein-sequence, which was derived from the early Wuhan strain and published in January 2020 [Lu et al., Lancet, 2020. 395(10224): p. 565-574.].

S protein-based vaccines leave room for improvement for several reasons:
i) High SARS-CoV-2 nAb titer as a single blood parameter is not predictive of recovery from COVID-19. In fact, robust nAb activity in the blood does not protect against progression towards severe disease. In contrast, SARS-CoV-2-specific T cell responses are linked to milder COVID-19. Moreover, SARS-CoV-2 specific T cell responses without seroconversion have been reported in asymptomatic contacts of SARS-CoV-2 infected individuals. Taken together, antibodies alone are not able to prevent virus spread and an efficient T cell response is key for control and clearance of an ongoing infection.
ii) In most cases of SARS-CoV and MERS infections, antiviral antibodies and memory B cell responses quickly vanish and facilitate immune escape. In a significant proportion of convalescent COVID-19 patients, nAb levels are relatively low and start to decrease within 2-3 months after infection. Indeed, insights from infections with common cold coronaviruses suggest that humoral immunity after coronavirus infection is short-lasting. This also applies for SARS-CoV-2. In conclusion, focusing vaccination strategies on induction of nAbs may provide protection only for a limited time.
iii) Most importantly, the antigenic evolution of the S protein erodes the protective effect of nAbs. In comparison to other RNA viruses, coronaviruses such as SARS-CoV-2 only slowly acquire substitutions due to a proofreading function of the RNA dependent RNA polymerase (RdRp). Nevertheless, SARS-CoV-2 variants such as Alpha (previously B.1.1.7 , first detected in the UK), Beta (previously B.1.351, first detected in South Africa), Gamma (previously P.1, first detected in Brazil), and Delta (previously B.1.617.2, first detected in India) emerged recently. They are more transmissible and probably also cause more severe illness. These variants harbor mutations in the S protein that confer resistance to experimental entry inhibitors and nAbs. The Delta variant, which currently outcompetes all other variants, shows higher replication and more efficient S-protein-mediated entry as compared to the Alpha variant. Alpha is refractory to most nAbs targeting the NTD and relatively resistant to nAbs binding to the RBD of the S protein. Sera from convalescent or vaccinated individuals poorly neutralized the Beta variant or chimeric strains containing the S protein of Beta or Gama. In fact, nAbs induced by infection or vaccination select S protein mutations that help SARS-CoV-2 to escape the immune response. Similarly, common cold coronaviruses have been shown to undergo adaptive evolution in S protein regions to evade nAbs. Recent data suggest that recurrent deletions in the S protein drive antibody escape. In contrast to substitutions, deletions are not corrected by proofreading function and accelerate antigenic evolution of SARS-CoV-2. Taken together, convalescent and vaccinated individuals could become susceptible to infection by emerging SARS-CoV-2 variants.

In conclusion, rapid decay of nAb levels, lack of broad SARS-CoV-2 specific T cell responses, and antigen evolution all threaten the success of vaccination campaigns using S protein-based COVID-19 vaccines.

Based on the above-mentioned state of the art, the objective of the present invention is to provide means and methods to facilitate long lasting, cross-strain immune responses against SARS-CoV-2 infection through vaccination. This objective is attained by the subject-matter of the independent claims of the present specification, with further advantageous embodiments described in the dependent claims, examples, figures and general description of this specification.

### Summary of the Invention

Waning antibody levels and emergence of novel SARS-CoV-2 variants may facilitate reinfections of convalescent individuals or breakthrough infections in the vaccinated population. Prophylactic vaccines that induce broad, polyfunctional, and long-lasting T cell responses against SARS-CoV-2 could solve the problem. Moreover, acute COVID-19 typically starts with mild symptoms 10 to 14 days before patients are admitted to the intensive care unit (ICU). Thus, there is a time window in which therapeutic T cell-inducing vaccines could be applied to prevent death due to acute respiratory distress syndrome (ARDS) and/or systemic inflammation. Finally, there are anecdotal reports that people suffering from Long-COVID-19 recover after being vaccinated. Long-COVID-19 could be due to incomplete viral clearance and boosting CD8+ T cells with a vaccine could help the immune system to get rid of the pathogen.

For generation of such vaccines, we have designed a consensus DNA sequence encoding all highly immunogenic and conserved T cell epitopes from structural proteins (S, E, M, N), nonstructural proteins (NSP) and accessory proteins of pandemic coronaviruses (PanCoVac). This multi-epitope vaccine is structured in such a way that the single T cell epitopes of a particular viral protein are strung like pearls on a necklace (epitope string). To allow efficient processing of the PanCoVac-encoded multi-epitope protein, the single T cell epitopes of a viral protein are separated by alanine spacers (epitope-alanine-strings). Moreover, the epitope-alanine-strings derived from the different viral proteins are linked by furin cleavage sites. Multi-epitope vaccines can induce a highly targeted immune responses and avoid unnecessary antigenic load, which may trigger either pathological immune responses or adverse effects. Due to the compact format of the PanCoVac-sequence, PanCoVac-based prophylactic or therapeutic vaccines can be easily generated by using currently available vaccine platforms such as mRNA delivered by LNPs or replication-incompetent adenoviral vectors.

In one aspect, the invention relates to a vaccine antigen amino acid sequence comprising
a. a nonstructural protein (NSP) epitope polypeptide sequence comprising a plurality of nonstructural protein T cell epitopes;
b. an accessory protein epitope polypeptide sequence comprising a plurality of accessory protein T cell epitopes;
c. an S (spike) protein epitope polypeptide sequence comprising a plurality of S protein T cell epitopes;
d. an E (envelope) protein epitope polypeptide sequence comprising a plurality of E protein T cell epitopes;
e. an M (membrane) protein epitope polypeptide sequence comprising a plurality of M protein T cell epitopes;
f. an N (nucleocapsid) protein epitope polypeptide sequence comprising a plurality of N protein T cell epitopes.

Each T cell epitope separated from another T cell epitope by an alanine stretch peptide.

A further aspect of the invention relates to a nucleic acid sequence encoding the vaccine antigen amino acid sequence according to the above aspect.

Another aspect of the invention relates to use of the vaccine antigen amino acid sequence or the nucleic acid sequence according to the invention for use as a vaccine to prevent or ameliorate symptoms of COVID-19 disease. Alternatively, the vaccine can be administered as a treatment of Long-COVID-19.

### Terms and definitions

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising," "having," "containing," and "including," and other similar forms, and grammatical equivalents thereof, as used herein, are intended to be equivalent in meaning and to be open-ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictates otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a," "or," and "the" include plural referents unless the context clearly dictates otherwise.

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic, and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

### Sequences

### General Biochemistry: Peptides, Amino Acid Sequences

The term *polypeptide* in the context of the present specification relates to a molecule consisting of 50 or more amino acids that form a linear chain wherein the amino acids are connected by peptide bonds. The amino acid sequence of a polypeptide may represent the amino acid sequence of a whole (as found physiologically) protein or fragments thereof. The term "polypeptides" and "protein" are used interchangeably herein and include proteins and fragments thereof. Polypeptides are disclosed herein as amino acid residue sequences.

The term *peptide* in the context of the present specification relates to a molecule consisting of up to 50 amino acids, in particular 8 to 30 amino acids, more particularly 8 to 15amino acids, that form a linear chain wherein the amino acids are connected by peptide bonds.

Amino acid residue sequences are given from amino to carboxyl terminus. Capital letters for sequence positions refer to L-amino acids in the one-letter code (Stryer, Biochemistry, 3^{rd} ed. p. 21). Lower case letters for amino acid sequence positions refer to the corresponding D- or (2R)-amino acids. Sequences are written left to right in the direction from the amino to the carboxy terminus. In accordance with standard nomenclature, amino acid residue sequences are denominated by either a three letter or a single letter code as indicated as follows: Alanine (Ala, A), Arginine (Arg, R), Asparagine (Asn, N), Aspartic Acid (Asp, D), Cysteine (Cys, C), Glutamine (Gin, Q), Glutamic Acid (Glu, E), Glycine (Gly, G), Histidine (His, H), Isoleucine (Ile, I), Leucine (Leu, L), Lysine (Lys, K), Methionine (Met, M), Phenylalanine (Phe, F), Proline (Pro, P), Serine (Ser, S), Threonine (Thr, T), Tryptophan (Trp, W), Tyrosine (Tyr, Y), and Valine (Val, V).

The term *nucleic acid expression vector* in the context of the present specification relates to a plasmid, a viral genome or an RNA, which is used to transfect (in case of a plasmid or an RNA) or transduce (in case of a viral genome) a target cell with a certain gene of interest, or -in the case of an RNA construct being transfected- to translate the corresponding protein of interest from a transfected mRNA. For vectors operating on the level of transcription and subsequent translation, the gene of interest is under control of a promoter sequence and the promoter sequence is operational inside the target cell, thus, the gene of interest is transcribed either constitutively or in response to a stimulus or dependent on the cell's status. In certain embodiments, the viral genome is packaged into a capsid to become a viral vector, which is able to transduce the target cell.

### Detailed Description of the Invention

SARS-CoV and SARS-CoV-2 express multiple nonstructural and structural proteins including S protein, envelope (E) protein, membrane (M) glycoprotein, and nucleocapsid (N) phosphoprotein.

Upon viral infection, viral proteins are expressed and processed into small peptides by proteasome and other proteases. These peptides are then loaded into the peptide binding groove of major histocompatibility complex (MHC) molecules (human leukocyte antigen [HLA] in people), which comprise class I (MHC-I) or class II (MHC-II), and presented on the surface of the infected cells. These epitopes are subsequently recognized by T cells with complementary T cell receptors (TCR). CD4+ T cells express TCRs that interact with MHC-II-bound peptides. In contrast, TCRs of the CD8+ T cell population detect peptides bound to MHC-I.

Although the S protein is a source of CD4+ T cell epitopes, a large pool of predicted MHC-II binding epitopes is located in other viral proteins, predominantly in the N and M protein [35, 122]. Intriguingly, the M protein harbours many CD4+ T cell epitopes although it is a comparatively small multipass membrane protein [16, 117, 122, 140, 141]. It has been estimated that each convalescent individual recognizes ca. 19 CD4+ T cell epitopes on average [102]. CD8+ T cell responses are frequently driven by a few immunodominant MHC-I epitopes [117]. Intriguingly, a higher proportion of polyfunctional M/N-specific CD8+ T cells compared with S-specific CD8+ T cells were detected in individuals who had recovered from mild COVID-19 [126]. In fact, most epitopes that are shared by CD8+ T cells of COVID-19 patients are located outside the S protein [116, 123]. It is important to include epitopes from all structural proteins because there is a strong inverse correlation between the predicted MHC-I epitope load and mortality from SARS-CoV-2 [142]. At least 17 CD8+ T cell epitopes per convalescent individual are recognized on average [102]. Which of the potential T cell epitopes are actually targeted depends on the MHC alleles, which vary widely from person to person. This large variety of T cell epitopes makes it difficult for viruses to escape T cell recognition. In conclusion, the T cell response directed against SARS-CoV-2 seems to be much broader than that observed after infection with other RNA viruses such as SARS-CoV [143] and dengue [144, 145]. Thus, T cell epitopes not only from the S protein but also from other viral proteins such as the M and the N protein should be included in future vaccines against SARS-CoV-2 to induce a strong, broad and poly-functional T cell response that SARS-CoV-2 cannot escape.

The first-generation vaccines focused on induction of neutralizing antibodies against the viral S protein, a strategy that facilitates immune escape. In contrast, we want to generate vaccines that induce a broad, robust and polyfunctional T cell response against conserved SARS-CoV-2 epitopes with emphasis on CD8+ T cells. For this purpose, we have identified the conserved T cell epitopes in structural proteins (S, E, M, N) from both SARS-CoV, SARS-CoV-2 as well as the four endemic common cold coronaviruses by using freely available algorithms (such as NetMHCpan 4.1 and NetMHCII 2.3) [146]. The majority of the antiviral CD8+ T cells recognize epitopes within the S, N, and M proteins [122]. However, algorithms predicting peptides that are naturally presented often yield false-positive results, especially if peptides with low binding adffinity are regarded [147]. Thus, peptide-encoding sequences that are based purely on in silico analyses [148] are less suitable for vaccine design.

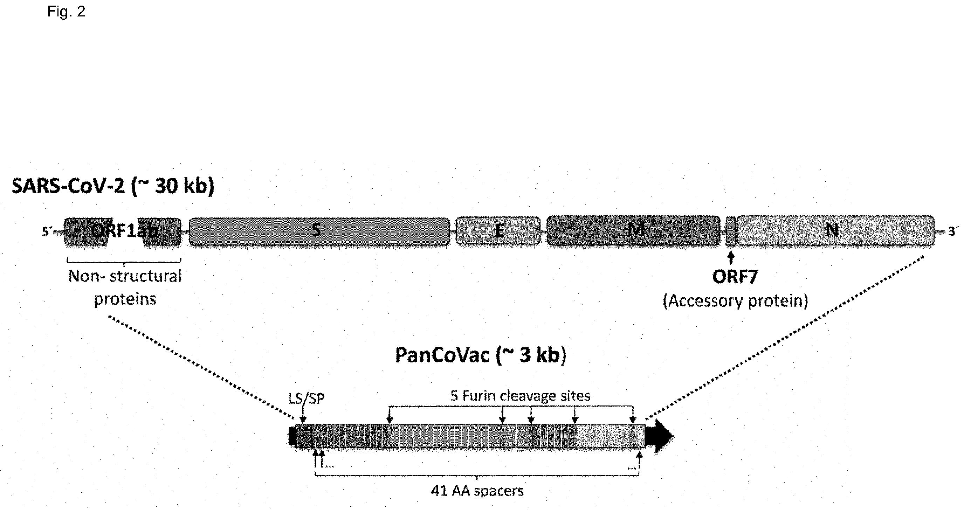

The PanCoVac sequence according to the invention includes predicted epitopes that are experimentally validated, that are conserved in SARS-CoV and SARS-CoV-2, and that show strong binding affinity to HLA class I and II [Altmann and Boyton, Science immunology, 2020. 5(49); Campbell et al., Prediction of SARS-CoV-2 epitopes across 9360 HLA class I alleles. BioRxiv, 2020.]. In fact, PanCoVac encodes all conserved HLA class I epitopes covering the important HLA class I supertypes (A1, A2, A3, A24, A26, B7, B8, B27, B39, B44, B58, B62). HLA supertypes are defined as HLA allelic variants that bind peptides possessing a particular HLA supermotif [Sette and Sidney, Immunogenetics, 1999. 50(3): p. 201-212]. We also codon-optimized the PanCoVac-sequence and modified it in such a way that it allows efficient processing and presentation of the PanCoVac protein in human cells. Usually, the residues flanking MHC-I epitopes determine the efficiency of processing by the proteasome system. This positional effect can be rescued by flanking epitopes with multiple alanines. For this reason, we separated the T cell epitopes within the same structural protein by double alanine (-AA) spacers. In addition, the resulting epitope-alanine-strings derived from the different structural proteins (S, E, M, M, and N protein) were linked by furin sites to allow cleavage of the PanCoVac-polyprotein into the single epitope-alanine-strings. In this way, PanCoVac reflects the broad T cell stimulatory capacity of SARS-CoV-2 and related coronaviruses in a compact form and can be easily delivered using different vaccine platforms (Figure 2). This is a major advantage compared to peptide vaccines, which are poorly immunogenic and require in vitro-synthesis of multiple peptides of 20-30 amino acids.

In one aspect, the invention relates to a vaccine antigen amino acid sequence comprising
a. a nonstructural protein epitope polypeptide sequence comprising, particularly consisting of, a plurality of nonstructural protein T cell epitopes, each nonstructural protein T cell epitope separated from another nonstructural protein T cell epitope by an alanine stretch peptide, wherein the plurality of nonstructural protein T cell epitopes comprises at least 10 sequences selected from SEQ ID NO 006, SEQ ID NO 007, SEQ ID NO 008, SEQ ID NO 009, SEQ ID NO 010, SEQ ID NO 011, SEQ ID NO 012, SEQ ID NO 013, SEQ ID NO 014, SEQ ID NO 015, SEQ ID NO 016, SEQ ID NO 017, SEQ ID NO 018,
b. an accessory protein epitope polypeptide sequence comprising, particularly consisting of, a plurality of nonstructural protein T cell epitopes, each nonstructural protein T cell epitope separated from another nonstructural protein T cell epitope by an alanine stretch peptide, wherein the plurality of accessory protein T cell epitopes comprises SEQ ID NO 019 and SEQ ID NO 020;
c. an S (spike) protein epitope polypeptide sequence comprising, particularly consisting of, a plurality of S protein T cell epitopes, each S protein T cell epitope separated from another S protein T cell epitope by an alanine stretch peptide, wherein the plurality of S protein T cell epitopes comprises at least 12 sequences selected from SEQ ID NO 021 SEQ ID NO 022, SEQ ID NO 023, SEQ ID NO 024, SEQ ID NO 025, SEQ ID NO 026, SEQ ID NO 027, SEQ ID NO 028, SEQ ID NO 029, SEQ ID NO 030, SEQ ID NO 031, SEQ ID NO 032, SEQ ID NO 033, SEQ ID NO 034, SEQ ID NO 035, SEQ ID NO 036, SEQ ID NO 037;
d. an E (envelope) protein epitope polypeptide sequence comprising, particularly consisting of, a plurality of E protein T cell epitopes, each E protein T cell epitope separated from another E protein T cell epitope by an alanine stretch peptide, wherein the plurality of E protein T cell epitopes comprises the sequences SEQ ID NO 038 and SEQ ID NO 039;
e. an M (membrane) protein epitope polypeptide sequence comprising, particularly consisting of, a plurality of M protein T cell epitopes, each M protein T cell epitope separated from another M protein T cell epitope by an alanine stretch peptide, wherein the plurality of M protein T cell epitopes comprises at least 4 sequences selected from SEQ ID NO 040, SEQ ID NO 041, SEQ ID NO 042, SEQ ID NO 043, SEQ ID NO 044, SEQ ID NO 045;
f. an N (nucleocapsid) protein epitope polypeptide sequence comprising, particularly consisting of, a plurality of N protein T cell epitopes, each N protein T cell epitope separated from another N protein T cell epitope by an alanine stretch peptide, wherein the plurality of N protein T cell epitopes comprises at least 6 sequences selected from SEQ ID NO 046, SEQ ID NO 047, SEQ ID NO 048, SEQ ID NO 049, SEQ ID NO 050, SEQ ID NO 051, SEQ ID NO 052, SEQ ID NO 053, SEQ ID NO 054.

In certain embodiments, each alanine stretch peptide independent of any other alanine stretch peptide is selected from AA and AAA.

In certain embodiments, the nonstructural protein epitope polypeptide sequence comprises 12, 13, 14, or 15 sequences selected from SEQ ID NO 006, SEQ ID NO 007, SEQ ID NO 008, SEQ ID NO 009, SEQ ID NO 010, SEQ ID NO 011, SEQ ID NO 012, SEQ ID NO 013, SEQ ID NO 014, SEQ ID NO 015, SEQ ID NO 016, SEQ ID NO 017, SEQ ID NO 018, SEQ ID NO 019, SEQ ID NO 020.

In certain embodiments, the nonstructural protein epitope polypeptide sequence is split into a first nonstructural protein epitope polypeptide sequence and a second nonstructural protein epitope polypeptide sequence.

In certain particular embodiments, the first nonstructural protein epitope polypeptide sequence comprises 12 or 13 nonstructural protein T cell epitopes, and the second nonstructural protein epitope polypeptide sequence comprises two nonstructural protein T cell epitopes.

In certain more particular embodiments, the nonstructural protein T cell epitopes of the first nonstructural protein epitope polypeptide sequence are selected from SEQ ID NO 006, SEQ ID NO 007, SEQ ID NO 008, SEQ ID NO 009, SEQ ID NO 010, SEQ ID NO 011, SEQ ID NO 012, SEQ ID NO 013, SEQ ID NO 014, SEQ ID NO 015, SEQ ID NO 016, SEQ ID NO 017, SEQ ID NO 018, and the nonstructural protein T cell epitopes of the second nonstructural protein epitope polypeptide sequence are SEQ ID NO 019 and 020.

In certain embodiments, the nonstructural protein and accessory epitope polypeptide sequence comprises SEQ ID NO 004 and SEQ ID NO 005, particularly a first nonstructural protein epitope polypeptide sequence comprises or is SEQ ID NO 004 and a second nonstructural (accessory) protein epitope polypeptide sequence comprises or is SEQ ID NO 005.

In certain embodiments, the S protein epitope polypeptide sequence comprises 14, 15, 16, or 17 sequences selected from SEQ ID NO 021 SEQ ID NO 022, SEQ ID NO 023, SEQ ID NO 024, SEQ ID NO 025, SEQ ID NO 026, SEQ ID NO 027, SEQ ID NO 028, SEQ ID NO 029, SEQ ID NO 030, SEQ ID NO 031, SEQ ID NO 032, SEQ ID NO 033, SEQ ID NO 034, SEQ ID NO 035, SEQ ID NO 036, SEQ ID NO 037.

### Sequential position of epitopes within the epitope polypeptide sequences

The architecture of PanCoVac reflects the natural epitope sequence of viral structural proteins in a compact form. The inventors do not know whether the natural order of epitopes in the sequence can be changed without suffering a loss of T-cell stimulatory activity. Theoretically, even with only 12 epitopes, there are about 500 million different possible combinations. The importance of sequential position of each epitope cannot be verified experimentally. However, experimental evidence points toward that an epitope placed in the middle of the PanCoVac sequence is processed and presented almost as efficiently as an epitope inserted at the C-terminal end of PanCoVac. Therefore, the inventors assume that there are no substantial position effects.

The AA linkers are always positioned at the N-terminal and C-terminal ends of the T-cell epitopes. According to the inventors' bioinformatic evaluation (proteasome score, TAP score), the AA linker is the most favorable in terms of processing and presentation of the PanCoVac-encoded T cell epitopes. AAY and AAA linkers are expected to deliver a comparable result. Extending the linkers to 4 or more amino acids is not useful from the inventors' point of view, since the probability of unwanted neo-epitopes and cleavage sites increases with the length of the spacers. In addition, the construct would become unnecessarily longer, potentially leading to losses in stability and translatability of the PanCoVac mRNA.

In certain embodiments, the nonstructural NSP epitope polypeptide sequence comprises or is SEQ ID NO 057.

In certain embodiments, the S protein epitope polypeptide sequence comprises or is SEQ ID NO 058.

In certain embodiments, the M protein epitope polypeptide sequence comprises 5 or 6 sequences selected from SEQ ID NO 040, SEQ ID NO 041, SEQ ID NO 042, SEQ ID NO 043, SEQ ID NO 044, SEQ ID NO 045.

In certain embodiments, the M protein epitope polypeptide sequence comprises or is SEQ ID NO 059.

In certain embodiments, the N protein epitope polypeptide sequence comprises 7, 8 or 9 sequences selected from SEQ ID NO 046, SEQ ID NO 047, SEQ ID NO 048, SEQ ID NO 049, SEQ ID NO 050, SEQ ID NO 051, SEQ ID NO 052, SEQ ID NO 053, SEQ ID NO 054.

In certain embodiments, the N protein epitope polypeptide sequence comprises or is SEQ ID NO 060.

In certain embodiments, each protein epitope polypeptide sequence is separated from its neighboring protein epitope polypeptide sequence by a furin cleavage peptide sequence, particularly wherein said furin cleavage peptide sequence is RGRKRRS (SEQ ID NO 055)

In certain embodiments, the vaccine antigen amino acid sequence according to any one of the preceding embodiments or a combination thereof, further comprises a leader sequence/signal peptide selected from an IgE heavy chain sequence and a tPA gene sequence forming the N terminal end of the vaccine antigen amino acid sequence.

The IgE leader sequence is known to improve translation of the pan vaccine mRNA [Vijayachari et al., Human vaccines & immunotherapeutics, 2015. 11(8): p. 1945-1953; Yan et al., Molecular Therapy, 2007. 15(2): p. 411-421]. It further facilitates introduction of the PanCoVac protein into the secretory pathway. The latter is necessary for furin to recognize and cleave the Furin cleavage sites incorporated in the PanCoVac sequence. Furin is a cellular endoprotease localised in the trans-Golgi network (TGN) - a late Golgi structure responsible for sorting secretory pathway proteins to their final destinations, including the cell surface, endosomes, lysosomes and secretory granules. Although furin is mainly localised in the TGN, it shuttles between this sorting compartment, the cell surface and early endosomes. It mediates the maturation of many proproteins by cleavage at precise junctions. Importantly, in this context, furin can generate immunologically important epitopes independently of TAP and the proteasome. Without wanting to be bound by their scientific reasoning, the inventors therefore assume that furin plays a role in the successful processing of the PanCoVac protein.

The terms leader sequence and signal sequence are used synonymously herein, whereas the terms generally refer to different biological phenomena (see (Mølhøj and Degan, Nature Biotechnology, 2004. 22(12): p. 1502-1502). Many other leader or signal sequences may be employed (see Table S1 in Haryadi et al., PLOS ONE, 2015. 10(2): p. e0116878).

In certain embodiments, the vaccine antigen amino acid sequence is or comprises SEQ ID NO 001.

A further aspect of the invention relates to a nucleic acid sequence encoding the vaccine antigen amino acid sequence according to the above aspect in any one of the preceding embodiments. In a particular embodiment, the nucleic acid sequence comprises or consists of SEQ ID NO 003.

In certain embodiments, the nucleic acid sequence further comprises a promoter sequence operably linked to said nucleic acid sequence.

In certain embodiments, the nucleic acid sequence is comprised in a viral vector. This can be an RNA virus, particularly an RNA+ or RNA- virus, or a single- or double stranded DNA virus. Particular embodiments include primate adenoviruses.

In certain embodiments, the nucleic acid sequence is an RNA sequence. The RNA sequence can be contained in a virus particle, or can be a "naked" messenger RNA (mRNA) formulated for injection, for example as part of a liposome formulation.

In certain particular embodiments, the nucleic acid sequence is an mRNA.

In certain embodiments, the nucleic acid sequence is a DNA sequence, for example a plasmid or minicircle comprising the antigen encoding sequence under control of a promoter operable in a human cell, or as part of a DNA virus.

A particular set of embodiment relates to replication-defective adenoviral particles comprising the nucleic acid sequence encoding the antigen polypeptide according to any of the previous embodiments.

Another aspect of the invention relates to a vaccine antigen amino acid sequence according to any one of the preceding embodiments, or the nucleic acid sequence according to any one of the preceding embodiments, for use as a vaccine to prevent or ameliorate symptoms of Covid-19 disease. Alternatively, the vaccine can be administered as a treatment of Long-Covid-19.

SARS-CoV and SARS-CoV-2 share 79.6 % sequence identity. The PanCoVac-encoded amino acid sequence is highly conserved in pathogenic coronaviruses. This is illustrated by the alignment of PanCoVac with SARS-CoV (isolate Tor2) and SARS-CoV-2 (isolate Wuhan-Hu-1) as well as the variants B.1.7.7 and P.1 (not shown). The DNA sequence of PanCoVac is shown in SEQ ID NO 001.

### Method of Manufacture and Method of Treatment according to the invention

The invention further encompasses, as an additional aspect, the use of an amino acid or nucleic acid sequence as identified herein for use in a method of manufacture of a medicament for the prevention of SARS-CoV-2 infection or the treatment of its associated disease (COVID-19).

Similarly, the invention encompasses methods of treatment of a patient at risk of SARS-CoV-2 infection or its associated disease (COVID-19). This method entails administering to the patient an effective amount of an amino acid or nucleic acid sequence as identified herein as specified in detail herein.

Wherever alternatives for single separable features are laid out herein as "embodiments", it is to be understood that such alternatives may be combined freely to form discrete embodiments of the invention disclosed herein.

The invention is further illustrated by the following examples and figures, from which further embodiments and advantages can be drawn. These examples are meant to illustrate the invention but not to limit its scope.

### Description of the Figures

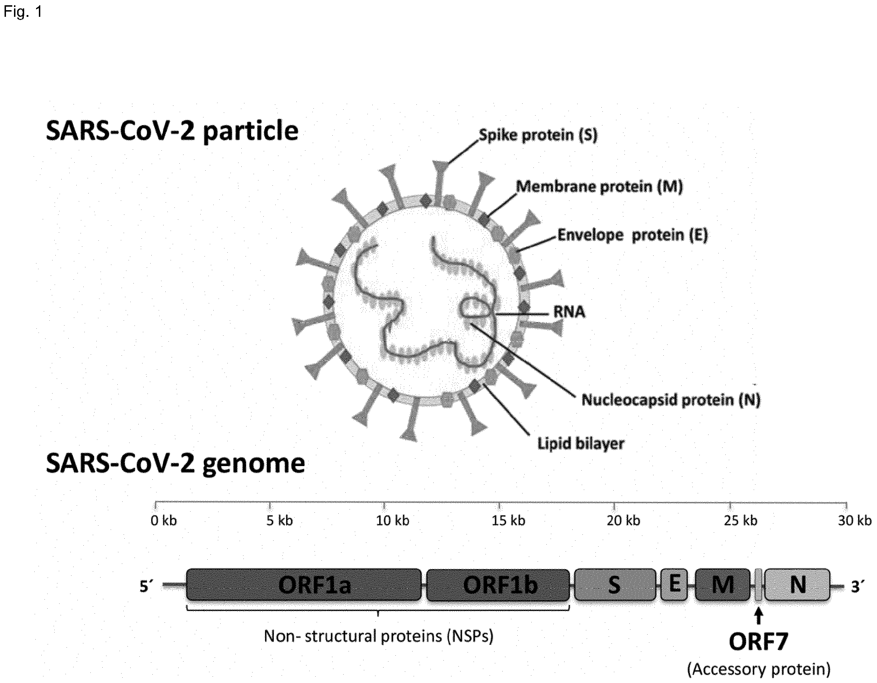

- Fig. 1: shows the schematic representation of the coronavirus genomic organisation. Coronaviruses form enveloped and spherical particles of 100 -160nm in diameter. They contain a positive-sense, single-stranded RNA (ssRNA) genome of 27-32 kb in size. The 5'-terminal two-thirds of the genome (ORF1 ab) encodes pp1ab. This polyprotein is further cleaved into 16 non-structural proteins that are involved in genome transcription and replication (not shown). The 3' terminus encodes structural proteins, including envelope glycoproteins spike (S), envelope (E), membrane (M) and nucleocapsid (N). In addition to the genes encoding structural proteins, there are accessory genes that are species-specific and dispensable for virus replication (e.g. ORF7).
- Fig. 2: shows a diagrammatic illustration of the PanCoVac design. The PanCoVac sequence is a compact form of the conserved T cell epitopes found in the structural proteins (S, E, M, N), non-structural proteins (ORF1ab), and an accessory protein (ORF7). The T cell epitopes within the same protein are separated by alanine (-AA) spacers to generate an epitope-alanine-string. The epitope-alanine-strings from different proteins are linked by furin sites to allow cleavage of the PanCoVac-polyprotein into single epitope-alanine-strings.

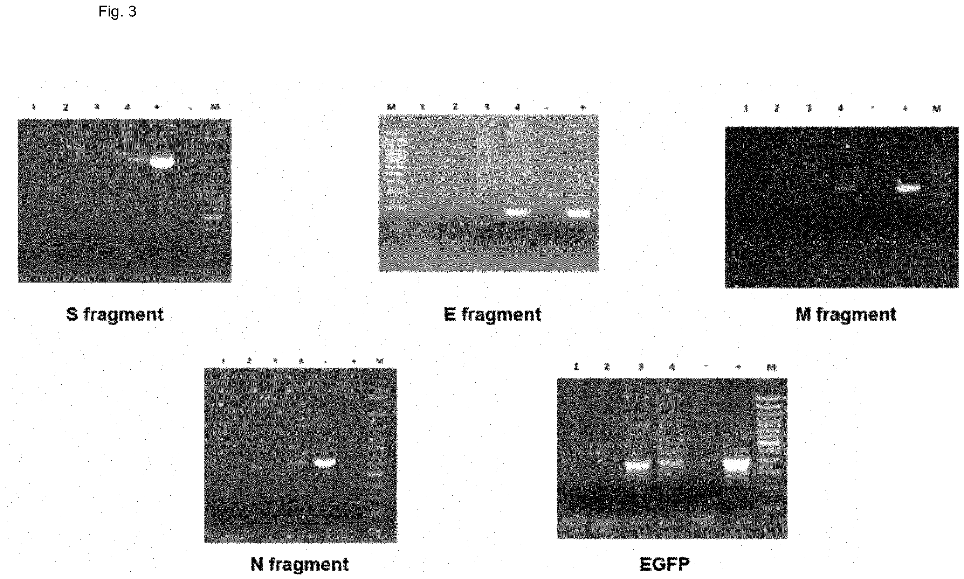

- Fig. 3: shows RT-PCR of PanCoVac fragments extracted from transduced U251 cells. RNA was harvested from cells transduced with empty and PanCoVac-expressing lentivirus (LV) and used to synthesize cDNA by reverse transcription. Lane 1 is LV empty - RNA, 2 is LV-PanCoVac - RNA, 3 is LV empty + RNA, 4 is LV-PanCoVac + RNA, negative (-) and positive controls (+). M is 100 bp ladder (Invitrogen).

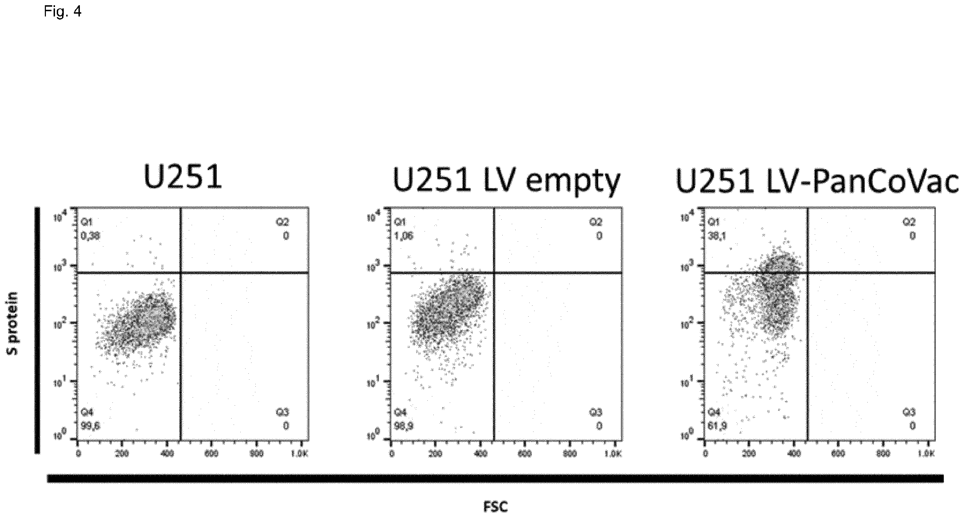

- Fig. 4: shows intracellular staining of PanCoVac for spike protein. U251 cells transduced with lentivirus using either an empty LeGO-iG2 transfer plasmid (U251 LV empty) or PanCoVac-containing plasmid (U251 LV-PanCoVac).

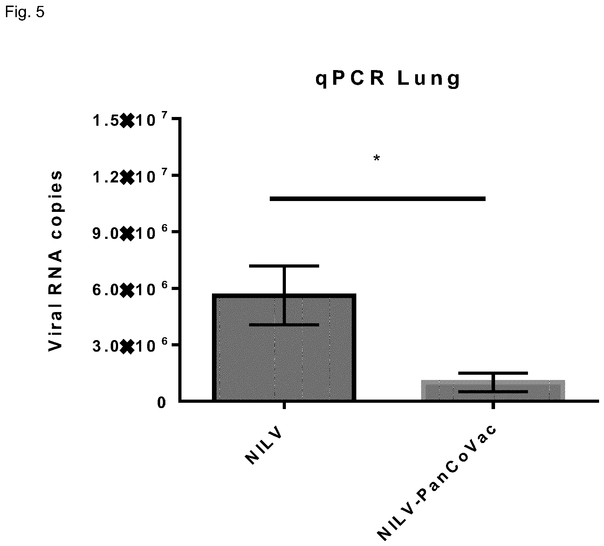

- Fig. 5: shows real-time PCR of SARS-CoV-2 E gene in a dwarf hamster model. The animals were immunized intranasal with 1×10⁸ virus particles of NILV (control) or NILV-PanCoVac. Afterwards, the animals were challenged with 1×10⁴ PFU of SARS-CoV-2. The graph shows the cumulative qPCR results of animals from 2 to 7 days post infection (dpi) (n=9).

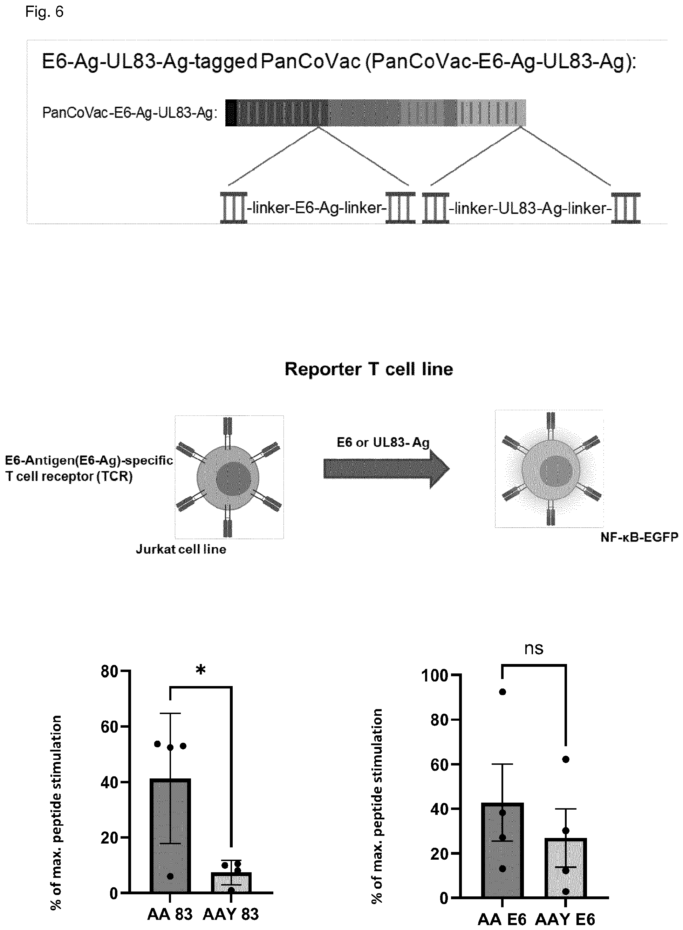

- Fig 6: shows the enhancement of antigen processing and presentation of PanCoVac by alanine (AA) linker and IgE leader sequence. For the optimal selection of peptide linker, two linkers (AA or AAY) were tested in two different versions of PanCovac. The HLA-A2 U251 cells were transfected with PanCoVac tagged with HPV16 E6 peptide (in the middle) and HCMV UL83 (at the C-terminus ) using different linker (AA or AAY). After 24 hours, the transfected cells were co-cultured with specific Jurkat reporter cell lines. The EGFP signal corresponding to NF-κB activation was measured by FACS. Stimulation of reporter cells is given as percentage of maximal peptide stimulation, i.e., stimulation of reporter cells incubated with peptide pulsed U251cells. The error bars represent the mean ± SEM. ns: not significant and *P < 0.05; unpaired t-test.

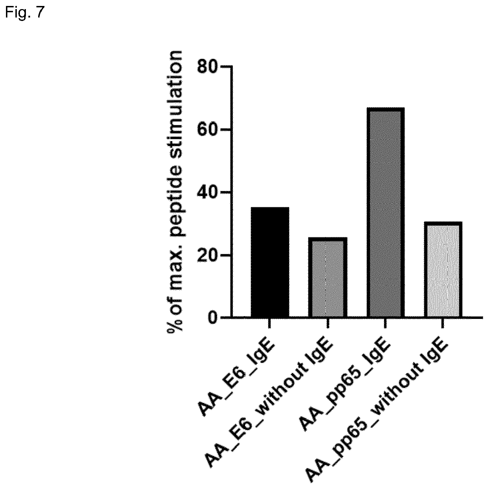

- Fig 7: shows the effect of the IgE Leader sequence on antigen processing and presentation. For enhancing the antigen presentation, the PanCoVac with AA linkers was synthesized with or without the IgE leader sequence. PanCoVac was tagged with HPV16 E6 peptide (in the middle) and HCMV UL83 (at the C-terminus ) using AA linker. After 24 hours, the transfected cells were co-cultured with specific Jurkat reporter cell lines. The EGFP signal corresponding to NF-κB activation was measured by FACS. Stimulation of reporter cells is given as percentage of maximal peptide stimulation, i.e., stimulation of reporter cells incubated with peptide pulsed U251cells.

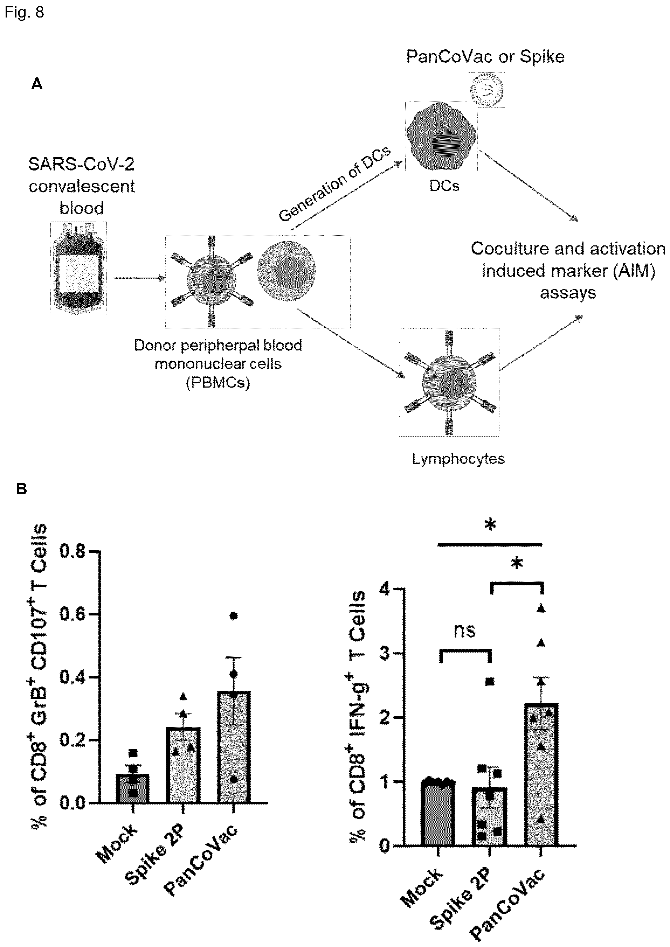

- Fig.8: Re-stimulation of primary T-cells by autologous APCs. The PBMCs from convalescent SARS-CoV-2 blood donors were isolated and the dendritic cells (DCs) were generated and untransfected (Mock) or transfected with mRNA (PanCoVac or SRAS-CoV-2 spike). The spike was in the prefusion form (2p). The transfected cells were afterwards cocultured with the autologous T cell and CD8+ T cell were stained for IFN-γ release, granzyme B (GrB) and CD107a . PanCovac showed stronger CD8+ T cell stimulation than Spike 2p, especially for IFN-γ. The error bars represent the mean ± SEM.

### Examples

### Example 1:

For preclinical testing of PanCoVac, the inventors employed a non-integrating lentiviral vector (NILV) as a vaccine platform (NILV-PanCoVac) [Apolonia, Viruses, 2020. 12(10).; Ku et al., Cell host & microbe, 2021. 29(2): p. 236-249.e6]. Lentiviral vectors can efficiently deliver antigens to proliferating and non-dividing cells such as dendritic cells and induce strong and long-lasting adaptive immunity. Similar to integrative lentiviral platforms, NILVs display strong vaccine efficacy but do not cause insertional mutagenesis. It has been shown that S protein-expressing NILVs inhibit lung deleterious injury in small animal models of COVID-19. As another advantage, NILVs are not restricted by pre-existing immunity because they are pseudotyped with the envelope glycoprotein of vesicular stomatitis virus (VSV-G), to which human beings are rarley exposed. Importantly, the safety of NILVs in humans has been shown in a phase 1 trial testing a HIV-1 vaccine.

In order to demonstrate successful transcription of PanCoVac sequence areas encoding epitopes from structural viral proteins the U251 cell line was transduced with lentiviral vector expressing PanCoVac. Subsequently, the mRNA was isolated and used for reverse transcriptase PCR (RT-PCR) to demonstrate successful transcription of PanCoVac sequence areas encoding epitopes from structural viral proteins in U251 cells. We sought to amplify fragments of the all structural proteins (S, E, M and N) by PCR using primers specific to each protein coding component (Fig. 3). The expression of S protein in transduced U251 cells with PanCoVac-expressing lentivirus was quantified by flow cytometry using PE-labelled secondary antibodies against anti-S protein primary antibodies (Fig. 4). We have also tested the potential protective capacity of NILV-PanCoVac in dwarf hamster model. The hamsters were inoculated with a non-PanCoVac expressing non-integrating Lentivirus particles (NILV) or with NILV-PanCoVac with a single dose of 1×10⁸ virus particles intranasal. After three weeks, the animals were challenged with a lethal dose of SARS-CoV-2 (1×10⁴ PFU) intranasal. Nasal swabs were collected from the infected animals for virus quantification using q-PCR. The animals were randomized and 3 animals were sacrificed at different timed points, the lungs were isolated and the virus was quantified using q-PCR (Fig. 5).

### Sequences

SEQ ID NO 001: PanCoVac amino acid sequence including leader sequence 
MDWTWILFLVAAATRVHSRPDTRYVLAATPKYKFVRIAAFLLPSLATVAAKLWAQCVQLAATEAFEKMVSLAAAL
WEIQQVVAACTDDNALAYYAAVTDTPKGPKAATEVPANSTVLAADLKGKYVQIAALSFKELLVYAAIPRRNVATL
AAMMISAGFSLRGRKRRSMFIFLLFLTAAKGIYQTSNFRVAAVRFPNITNLCPFGEVFNATRFASVYAWNRKRSN
CVADYSVLYNSAAGYQPYRVVVLSFELLAACVNFNFNGLTGTGVLTAANLLLQYGSFCTQLNRALAASFIEDLLF
NKVTLADAGFAAARDLICAQKFNGLTVLPPLLAAGWTFGAGAALQIPFAMQMAYRFNGIGVTQNVLYENQKAAAL
GKLQDVVNQNAQALNTLVKQLSSNFGAISSVLNDILSRLDKVEAEVQIDRLITGRLQSLQTYVTQQLIRAAEIRA
SANLAATKMSECVLGQSKRVDFCGKGYHLMSAAAPHGVVFLHVTYVAAQIITTDNTFVSGNCDVVIGIAANTVYD
PLQPELDSFKEELDKYFKNHTSPDVDLGDISGINASVVNIQKEIDRLNEVAKNLNESLIDLQELGKYEQYIKWPW
YIAAWLGFIAGLIAIVMVTIAACCKFDEDDSEPVLKGVKLHYRGRKRRSMYSFVSEETGTLIVNSVLLFLAFVVF
 
LLVTLAILTALRLCAYCCNIVNVSLVKAAYVYSRVKNLRGRKRRSNRNRFLYIIKLAAFLWLLWPVTLACFVLAA
VYRINWAAASFRLFARTRSMWSFNPETNILLNVPLAAESELVIGAVIAAGRCDIKDLPKEITVATSRTLSYYKLG
ASQRVAGDAARYRIGNYKLNTRGRKRRSKQRRPQGLPNNTASWFTALTQHGKEAAFPRGQGVPINAALSPRWYFY
YLGTGPEAAAVLQLPQGTTLPKGFYAEGSRGGSQASSRSSSRSRAAALALLLLDRLNQLESKAAKGQQQQGQTVT
KKSAAEASKKPRQKRTATKAAYNVTQAFGRRGPEQTQGNFAALIRQGTDYKHWPQIAQFAPSASAFFGMSRIGME
VTPSGTWLTYAAVILLNKHIDAYKTFPPTEPKKDKKKKADETRGRKRRSTYEGNSPFHPLADNKFALAARARSVS
PKL
SEQ ID NO 002: PanCoVac amino acid sequence without leader sequence

RPDTRYVLAATPKYKFVRIAAFLLPSLATVAAKLWAQCVQLAATEAFEKMVSLAAALWEIQQVVAACTDDNALAY
YAAVTDTPKGPKAATEVPANSTVLAADLKGKYVQIAALSFKELLVYAAIPRRNVATLAAMMISAGFSLRGRKRRS
MFIFLLFLTAAKGIYQTSNFRVAAVRFPNITNLCPFGEVFNATRFASVYAWNRKRSNCVADYSVLYNSAAGYQPY
RVVVLSFELLAACVNFNFNGLTGTGVLTAANLLLQYGSFCTQLNRALAASFIEDLLFNKVTLADAGFAAARDLIC
AQKFNGLTVLPPLLAAGWTFGAGAALQIPFAMQMAYRFNGIGVTQNVLYENQKAAALGKLQDVVNQNAQALNTLV
KQLSSNFGAISSVLNDILSRLDKVEAEVQIDRLITGRLQSLQTYVTQQLIRAAEIRASANLAATKMSECVLGQSK
RVDFCGKGYHLMSAAAPHGVVFLHVTYVAAQIITTDNTFVSGNCDVVIGIAANTVYDPLQPELDSFKEELDKYFK
NHTSPDVDLGDISGINASVVNIQKEIDRLNEVAKNLNESLIDLQELGKYEQYIKWPWYIAAWLGFIAGLIAIVMV
TIAACCKFDEDDSEPVLKGVKLHYRGRKRRSMYSFVSEETGTLIVNSVLLFLAFVVFLLVTLAILTALRLCAYCC
NIVNVSLVKAAYVYSRVKNLRGRKRRSNRNRFLYIIKLAAFLWLLWPVTLACFVLAAVYRINWAAASFRLFARTR
SMWSFNPETNILLNVPLAAESELVIGAVIAAGRCDIKDLPKEITVATSRTLSYYKLGASQRVAGDAARYRIGNYK
LNTRGRKRRSKQRRPQGLPNNTASWFTALTQHGKEAAFPRGQGVPINAALSPRWYFYYLGTGPEAAAVLQLPQGT
TLPKGFYAEGSRGGSQASSRSSSRSRAAALALLLLDRLNQLESKAAKGQQQQGQTVTKKSAAEASKKPRQKRTAT
KAAYNVTQAFGRRGPEQTQGNFAALIRQGTDYKHWPQIAQFAPSASAFFGMSRIGMEVTPSGTWLTYAAVILLNK
HIDAYKTFPPTEPKKDKKKKADETRGRKRRSTYEGNSPFHPLADNKFALAARARSVSPKL

### Epitope-encoding sequences:

▪ Non-structural protein (NSP) epitopes
▪ Spike (S) protein epitopes
▪ Envelope (E) protein epitopes
▪ Membrane (M) protein epitopes
▪ Nucleocapsid (N) epitopes
▪ Accessory epitopes encoded by ORF7

Additional sequences regulating expression, transport, and processing:
▪ IgE leader sequence
▪ Double alanine spacers
▪ Furin cleavage sites
SEQ ID NO 003: PanCoVac nucleic acid sequence (this is a contiguous sequence)

| **IgE leader seq** | atg gac tgg acc tgg atc ctg ttt ctg gtg gcc get gcc aca aga gtg cac tca |
|---|---|
| **NSP** epitopes | aga ccc gat acc aga tac gtg ctg gcc gcc aca cct aag tac aag ttc gtc cgg atc gcc gcc ttc ctg ctg cct tct ttg gct aca gtg gcc gcc aaa ctg tgg gcc cag tgt gtt caa ctg gcc gct aca gag gcc ttc gag aag atg gtg tct ctg gct gcc gct ctg tgg gag atc cag caa gtg gtg gct gcc tgc acc gac gat aat gcc ctg gcc tat tat gcc gcc gtg aca gac aca cca aag ggc cct aaa gcc gcc aca ga g gtg cca gcc aat tct aca gtg ctg gca gcc gac ctg aag ggc aaa tac gtg cag a tc gcc gct ctg agc ttc aaa gaactg ctg gtg tac gcc gcc att cct cgg aga aat gtg gct acc ctg gct gcc atg atg atc agc gcc ggc ttt agc ctg |
| **Furin** cleavage site | aga ggc cgg aag cgg aga tcc |
| S epitopes | atg ttt atc ttt ctg ctg ttc ctg acc gcc gcc aag ggc atc tac cag acc agc aat ttc aga gtg gct gcc gtg cgg ttc ccc aac atc acc aat ctg tgc cct ttc ggc gag gtg tt c aat gcc aca  aga ttt gcc agc gtg tac gcc tgg aac cgg aag aga  agc aac tgc gtg gcc gac tac agc gtg ctg tac aat agc gcc gct ggc tac cag cct tac aga gtg gtg gtg ctg agc ttc gag ctg ctg gct gct tgc gtg aac ttc aac ttc aac ggc ctg acc ggc acc ggc gtt ctg aca gct gct aat ctg ctg ctg cag tac ggc agc ttc tgc acc cag ctg aat ag a gcc ctg gcc gcc  agc ttc atc gag gac ctg ctg ttc aac aaa gtg acc ctg gcc g at gcc gga ttt gcc gct gct agg gat ctg atc tgc gcc cag aag ttt aac gga ctg ac c gtg ctg cct cct ctg ctt gct gcc gga tgg aca ttt gga gct ggt gct gcc ctg cag a tc ccc ttc gct atg cag atg gcc tac cgg ttc aat ggc atc ggc gtg acc cag aat gt g ctg tac gag aac cag aag gcc gct gct ctg ggc aag ctg cag gat gtg gtc aac c ag aat gcc cag gct ctg aac acc ctg gtc aag cag ctg agc agc aac ttc ggc gcc atc agc tcc gtg ctg aac gac atc ctg agc cgg ctg gat aag gtg gaa gcc gag gtg cag att gac cgg ctg atc aca ggc aga ctg cag agc ctg cag acc tac gtg aca c ag cag ctg atc agg gcc gct gag att aga gcc tct gcc aac ctg gcc gca aca aag atg agc gag tgt gtg ctg ggc cag agc aag aga gtg gac ttt tgc ggc aag ggc ta c cac ctg atg tca gct gct gct cct cac ggc gtg gtg ttt ctg cac gtg aca tat gtg  gcc gct cag atc at c acc acc gac aac acc ttc gtg tcc ggc aac tgc gac gtg gtc att gga atc gcc gcc aat acc gtg tac g ac cct ctg cag cct gag ctg gac tcc ttc aaa gag gaa ctg gac aag tac ttc aag a ac cac aca agc ccc gac gtg gac ctg ggc gat atc tct ggc atc aat gcc agc gtc |
| | gtg aac atc cag aaa gag atc gac aga ctg aac gag gtg gcc aag aac ctg aac gag agc ctg atc gac ctg caa gag ctg ggg aag tac gag cag tac atc aag tgg c cc tgg tac att gcc gcc tgg ctg ggc ttt atc gcc gga ctg att gcc atc gtg atg gtc aca atc gcc gct  tgc tgc aag ttc gac gag gac gat agc gag cct gtg ctg aag gg c gtg aag ctg cac tac |
| **Furin cleavage site** | aga gga cgc aag agg cgg agc |
| **E epitopes** | atg tac agc ttt gtg tcc gag gaa acc ggc aca ctg atc gtg aat agc gtg ctc ctg tt c ctg gcc ttc gtc gtg ttc ctg ctg gtc aca ctg gcc atc ctg aca gcc ctg aga ctg t gc gcc tac tgc tgc aac atc gtg aac gtg tcc ctg gtt aag gcc gcc tac gtg tac agc aga gtg aag aac ctg |
| **Furin cleavage site** | aga gga cgc aag agg cgg agc |
| **M epitopes** | aac aga aac aga ttc ctg tac att atc aag ctg gct gcc ttc ctg tgg ttg ctg tgg cct gtg aca ctg gcc t gt ttt gtt ctg gcc gcc gtg tac cgg atc aat tgg gct gcc gcc tcc ttc cgg ctg ttt gcc aga acc aga tct atg tgg tcc ttc aat ccc gag aca aac atc ctg ctg aat gtg c cc ctg gca gct gag agc gag ctg gtt att gga gcc gtg atc gct gcc gga aga tgc g ac atc aag gat ctg ccc aaa gaa atc acc gtg gcc acc agc aga acc ctg agc tac tat aag ctg ggc gcc tct cag aga gtg gca ggc gac gct gcc aga tac aga atc gg c aac tac aag ctg aac acc |
| **Furin cleavage site** | aga ggc aga aaa agg cgg tcc |
| **N epitopes** | aaa cag aga agg cct cag ggc ctg cct aac aac acc gcc agc tgg ttt act gcc ctg act cag cac ggc aaa gaa gcc gcc ttt cct aga gga cag ggc gtg cca atc aac g cc gca ctg agc cct aga tgg tac ttc tac tac ctc ggc aca ggc cca gag gct gca g ct gtt ctg caa ctg cct cag gga acc aca ctg cct aag ggc ttt tac gcc gag ggc tct aga ggc gga agc cag gcc tct agc aga agc agc tct aga agc aga gcc gct gca ctg gca ttg ctg ctg ctg gac agg ctg aat cag ctg gaa agc aag gcc gct aag ggc cag cag caa cag gga cag acc gtg acc aag aag tct gcc gcc gag gcc agc aa g aag ccc aga cag aaa aga acc gcc acc aaa gcc gcc tac aat gtg acc cag gc c ttc ggc |
| | aga aga ggc cct gaa cag acc cag ggc aat ttc gct gcc ctg att aga cag ggc ac c gac tac aag cac tgg ccc cag att gcc cag ttt gcc cct agc gcc tct gcc ttt ttc g gc atg tct cgg atc ggc atg gaa gtg acc cct agc ggc aca tgg ctg aca tac gcc gct gtg att ctg ctg aac aag cac atc gac gcc tac aag aca ttc cct cca acc gag cct aag aag gac aag aag aag aag gcc gac gag aca |
| **Furin cleavage site** | cgc gga aga aag cgg aga agc |
| **Accessory epitopes** | acc tac gag ggc aac agc cct ttt cac cct ctg gcc gat aac aag ttc gcc ctc gct gct aga gcc aga agc g tg tca cct aaa ctg taa |

SEQ ID NO 004: First nonstructural protein epitope polypeptide sequence (located before the first furin cleavage site) 
RPDTRYVLAATPKYKFVRIAAFLLPSLATVAAKLWAQCVQLAATEAFEKMVSLAAALWEIQQVVAA
CTDDNALAYYAAVTDTPKGPKAATEVPANSTVLAADLKGKYVQIAALSFKELLVYAAIPRRNVATL
AAMMISAGFSL
 SEQ ID NO 005: Accessory protein epitope polypeptide sequence (located after the last furin cleavage site)
TYEGNSPFHPLADNKFAL**AA**RARSVSPKL
▪ NSP epitopes

| | |
|---|---|
| SEQ ID NO 006 | RPDTRYVL |
| SEQ ID NO 007 | TPKYKFVRI |
| SEQ ID NO 008 | FLLPSLATV |
| SEQ ID NO 009 | KLWAQCVQL |
| SEQ ID NO 010 | TEAFEKMVSL |
| SEQ ID NO 011 | ALWEIQQVV |
| SEQ ID NO 012 | CTDDNALAYY |
| SEQ ID NO 013 | VTDTPKGPK |
| SEQ ID NO 014 | TEVPANSTVL |
| SEQ ID NO 015 | DLKGKYVQI |
| SEQ ID NO 016 | LSFKELLVY |
| SEQ ID NO 017 | IPRRNVATL |
| SEQ ID NO 018 | MMISAGFSL |

▪ Accessory protein epitopes

| | |
|---|---|
| SEQ ID NO 019 | TYEGNSPFHPLADNKFAL |
| SEQ ID NO 020 | RARSVSPKL |

▪ S epitopes

| | |
|---|---|
| SEQ ID NO 021 | MFIFLLFLT |
| SEQ ID NO 022 | KGIYQTSNFRV |
| SEQ ID NO 023 | VRFPNITNLCPFGEVFNATRFASVYAWNRKRSNCVADYSVLYNS |
| SEQ ID NO 024 | VRQIAPGQTG |
| SEQ ID NO 025 | IADYNYKLPDDF |
| SEQ ID NO 026 | GYQPYRVWLSFELL |
| SEQ ID NO 027 | CVNFNFNGLTGTGVLT |
| SEQ ID NO 028 | NLLLQYGSFCTQLNRAL |
| SEQ ID NO 029 | SFIEDLLFNKVTLADAGF |
| SEQ ID NO 030 | ARDLICAQKFNGLTVLPPLL |
| SEQ ID NO 031 | GWTFGAGAALQIPFAMQMAYRFNGIGVTQNVLYENQK |
| SEQ ID NO 032 | ALGKLQDVVNQNAQALNTLVKQLSSNFGAISSVLNDILSRLDKVEAEVQID RLITGRLQSLQTYVTQQLIRAAEIRASANLAATKMSECVLGQSKRVDFCGKGYHLM S |
| SEQ ID NO 033 | APHGWFLHVTYV |
| SEQ ID NO 034 | QIITTDNTFVSGNCDWIGI |
| SEQ ID NO 035 | NTVYDPLQPELDSFKEELDKYFKNHTSPDVDLGDISGINASVVNIQKEIDR LNEVAKNLNESLIDLQELGKYEQYIKWPWYI |
| SEQ ID NO 036 | WLGFIAGLIAIVMVTI |
| SEQ ID NO 037 | CCKFDEDDSEPVLKGVKLHY |

▪ E epitopes

| | |
|---|---|
| SEQ ID NO 038 | MYSFVSEETGTLIVNSVLLFLAFWFLLVTLAILTALRLCAYCCNIVNVSLVK |
| SEQ ID NO 039 | YVYSRVKNL |

▪ M epitopes

| | |
|---|---|
| SEQ ID NO 040 | NRNRFLYIIKL |
| SEQ ID NO 041 | FLWLLWPVTLACFVLAAVYRINW |
| SEQ ID NO 042 | ASFRLFARTRSMWSFNPETNILLNVPL |
| SEQ ID NO 043 | ESELVIGAVI |
| SEQ ID NO 044 | GRCDIKDLPKEITVATSRTLSYYKLGASQRVAGD |
| SEQ ID NO 045 | RYRIGNYKLNT |

▪ N epitopes

| | |
|---|---|
| SEQ ID NO 046 | KQRRPQGLPNNTASWFTALTQHGKE |
| SEQ ID NO 047 | FPRGQGVPIN |
| SEQ ID NO 048 | LSPRWYFYYLGTGPEA |
| SEQ ID NO 049 | VLQLPQGTTLPKGFYAEGSRGGSQAS3RSSSR3R |
| SEQ ID NO 050 | ALALLLLDRLNQLESK |
| SEQ ID NO 051 | KGQQQQGQTVTKKSAAEASKKPRQKRTATK |
| SEQ ID NO 052 | YNVTQAFGRRGPEQTQGNF |
| SEQ ID NO 053 | LIRQGTDYKHWPQIAQFAPSASAFFGMSRIGMEVTPSGTWLTY |
| SEQ ID NO 054 | VILLNKHIDAYKTFPPTEPKKDKKKKADET |

▪ Furin cleavage sites

| | |
|---|---|
| SEQ ID NO 055 | RGRKKRS |

▪ IgE leader sequence: SEQ ID NO 056
SEQ ID NO 056 MDWTWILFLVAAATRVHS
SEQ ID NO 057 NSP epitope polypeptide sequence 
RPDTRYVLAATPKYKFVRIAAFLLPSLATVAAKLWAQCVQLAATEAFEKMVSLAAALWEIQQVVAACTD
DNALAYYAAVTDTPKGPKAATEVPANSTVLAADLKGKYVQIAALSFKELLVYAAIPRRNVATLAAMMIS
AGFSL
SEQ ID NO 058 S protein epitope polypeptide sequence: 
MFIFLLFLTAAKGIYQTSNFRVAAVRFPNITNLCPFGEVFNATRFASVYAWNRKRSNCVADYSVLYNSA
AGYQPYRVVVLSFELLAACVNFNFNGLTGTGVLTAANLLLQYGSFCTQLNRALAASFIEDLLFNKVTLA
DAGFAAARDLICAQKFNGLTVLPPLLAAGWTFGAGAALQIPFAMQMAYRFNGIGVTQNVLYENQKAAAL
GKLQDVVNQNAQALNTLVKQLSSNFGAISSVLNDILSRLDKVEAEVQIDRLITGRLQSLQTYVTQQLIR
AAEIRASANLAATKMSECVLGQSKRVDFCGKGYHLMSAAAPHGVVFLHVTYVAAQIITTDNTFVSGNCD
VVIGIAANTVYDPLQPELDSFKEELDKYFKNHTSPDVDLGDISGINASVVNIQKEIDRLNEVAKNLNES
LIDLQELGKYEQYIKWPWYIAAWLGFIAGLIAIVMVTIAACCKFDEDDSEPVLKGVKLHY.
SEQ ID NO 059 M protein epitope polypeptide sequence: 
NRNRFLYIIKLAAFLWLLWPVTLACFVLAAVYRINWAAASFRLFARTRSMWSFNPETNILLNVPLAAESE
LVIGAVIAAGRCDIKDLPKEITVATSRTLSYYKLGASQRVAGDAARYRIGNYKLNT
SEQ ID NO 060 N protein epitope polypeptide sequence:

KQRRPQGLPNNTASWFTALTQHGKEAAFPRGQGVPINAALSPRWYFYLGTGPEAAAVLQLPQGTTLPKG
FYAEGSRGGSQASSRSSSRSRAAALALLLLDRLNQLESKAAKGQQQQGQTVTKKSAAEASKKPRQKRTAT
KAAYNVTQAFGRRGPEQTQGNFAALIRQGTDYKHWPQIAQFAPSASAFFGMSRIGMEVTPSGTWLTYAAV
ILLNKHIDAYKTFPPTEPKKDKKKKADET.

## Claims

1. A vaccine antigen amino acid sequence comprising
a. a nonstructural protein epitope polypeptide sequence comprising, particularly consisting of, a plurality of nonstructural protein T cell epitopes, each nonstructural protein T cell epitope separated from another nonstructural protein T cell epitope by an alanine stretch peptide, wherein the plurality of nonstructural protein T cell epitopes comprises at least 10 sequences selected from SEQ ID NO 006 to SEQ ID NO 018;
b. an accessory protein epitope polypeptide sequence comprising, particularly consisting of, a plurality of nonstructural protein T cell epitopes, each nonstructural protein T cell epitope separated from another nonstructural protein T cell epitope by an alanine stretch peptide, wherein the plurality of accessory protein T cell epitopes comprises SEQ ID NO 019 and SEQ ID NO 020;
c. an S protein epitope polypeptide sequence comprising, particularly consisting of, a plurality of S protein T cell epitopes, each S protein T cell epitope separated from another S protein T cell epitope by an alanine stretch peptide, wherein the plurality of S protein T cell epitopes comprises at least 12 sequences selected from SEQ ID NO 021 to SEQ ID NO 037;
d. an E protein epitope polypeptide sequence comprising, particularly consisting of, a plurality of E protein T cell epitopes, each E protein T cell epitope separated from another E protein T cell epitope by an alanine stretch peptide, wherein the plurality of E protein T cell epitopes comprises the sequences SEQ ID NO 038 and SEQ ID NO 039;
e. an M protein epitope polypeptide sequence comprising, particularly consisting of, a plurality of M protein T cell epitopes, each M protein T cell epitope separated from another M protein T cell epitope by an alanine stretch peptide, wherein the plurality of M protein T cell epitopes comprises at least 4 sequences selected from SEQ ID NO 040 to SEQ ID NO 045;
f. an N protein epitope polypeptide sequence comprising, particularly consisting of, a plurality of N protein T cell epitopes, each N protein T cell epitope separated from another N protein T cell epitope by an alanine stretch peptide, wherein the plurality of N protein T cell epitopes comprises at least 6 sequences selected from SEQ ID NO 046 to SEQ ID NO 054.

2. The vaccine antigen amino acid sequence according to claim 1, wherein each alanine stretch peptide independent of any other alanine stretch peptide is selected from AA and AAA,
particularly wherein each alanine stretch peptide is AA.

3. The vaccine antigen amino acid sequence according to claim 1 or 2, wherein
a. the nonstructural protein epitope polypeptide sequence comprises 12, 13, 14, or 15 sequences selected from SEQ ID NO 006 to SEQ ID NO 018;
b. the S protein epitope polypeptide sequence comprises 14, 15, 16, or 17 sequences selected from SEQ ID NO 021 to SEQ ID NO 037;
c. the M protein epitope polypeptide sequence comprises 5 or 6 sequences selected from SEQ ID NO 040 to SEQ ID NO 045;
d. the N protein epitope polypeptide sequence comprises 7, 8 or 9 sequences selected from SEQ ID NO 046 to SEQ ID NO 054.

4. The vaccine antigen amino acid sequence according to any one of the preceding claims, wherein
a. the nonstructural protein epitope polypeptide sequence comprises SEQ ID NO 004;
b. the S protein epitope polypeptide sequence comprises or is SEQ ID NO 058;
c. the M protein epitope polypeptide sequence comprises or is SEQ ID NO 059;
d. the N protein epitope polypeptide sequence comprises or is SEQ ID NO 060.

5. The vaccine antigen amino acid sequence according to any one of the preceding claims, wherein the nonstructural protein epitope polypeptide sequence is split into a first nonstructural protein epitope polypeptide sequence and a second nonstructural protein epitope polypeptide sequence,
particularly wherein the first nonstructural protein epitope polypeptide sequence comprises 12 or 13 nonstructural protein T cell epitopes, and the second nonstructural protein epitope polypeptide sequence comprises two nonstructural protein T cell epitopes,
more particularly wherein the nonstructural protein T cell epitopes of the first nonstructural protein epitope polypeptide sequence are selected from SEQ ID NO 006 to SEQ ID NO 018,
and the nonstructural protein T cell epitopes of the second nonstructural protein epitope polypeptide sequence are SEQ ID NO 019 and 020.

6. The vaccine antigen amino acid sequence according to any one of the preceding claims, wherein each protein epitope polypeptide sequence is separated from its neighboring protein epitope polypeptide sequence by a furin cleavage peptide sequence, particularly wherein said furin cleavage peptide sequence is RGRKRRS (SEQ ID NO 055)

7. The vaccine antigen amino acid sequence according to any one of the preceding claims, wherein the vaccine antigen amino acid sequence is or comprises SEQ ID NO 001.

8. A nucleic acid sequence encoding the vaccine antigen amino acid sequence according to any one of the preceding claims,
particularly wherein the nucleic acid sequence comprises or consists of SEQ ID NO 003.

9. The nucleic acid sequence according to claim 8, further comprising a promoter sequence operably linked to said nucleic acid sequence.

10. The nucleic acid sequence according to claim 8 or 9, wherein the nucleic acid sequence is comprised in a viral vector.

11. The nucleic acid sequence according to claim 8 to 10, wherein the nucleic acid sequence is an RNA sequence, particularly wherein the nucleic acid sequence is an mRNA.

12. The nucleic acid sequence according to claim 8 to 10, wherein the nucleic acid sequence is a DNA sequence.

13. A replication-defective adenoviral particle comprising the nucleic acid sequence according to any one of claim 8 to 12.

14. The vaccine antigen amino acid sequence according to any one of the preceding claims 1 to 7, or the nucleic acid sequence according to any one of the preceding claims 8 to 13, for use
a. as a vaccine to prevent or ameliorate symptoms of Covid-19 disease;
b. as a treatment of Long-Covid-19.
